# EUROPÄISCHE PATENTSCHRIFT

(11) **EP 1 058 860 B1**
(45) Veröffentlichungstag und Bekanntmachung des Hinweises auf die Patenterteilung: **08.10.2003**
(21) Anmeldenummer: 98946249.4
(22) Anmeldetag: 30.07.1998
(51) Int. Cl.: G02B 23/24, A61B 1/00, A61B 1/24, A61B 1/04

(54) **KAMERA UND INSBESONDERE MODULARE DENTALKAMERA**
CAMERA AND, IN PARTICULAR, MODULAR DENTAL CAMERA
CAMERA ET NOTAMMENT CAMERA DENTAIRE MODULAIRE

(30) Priorität: 30.07.1997 DE 19732718
(43) Veröffentlichungstag der Anmeldung: 13.12.2000
(73) Patentinhaber: LINOS Photonics GmbH & Co. KG, 37081 Göttingen (DE)
(72) Erfinder: MATERN, Ulrich, D-82362 Weilheim (DE)
(74) Vertreter: Säger, Manfred, Dipl.-Ing.
(86) Internationale Anmeldenummer: DE9802178
(87) Internationale Veröffentlichungsnummer: WO99006871

(56) Entgegenhaltungen:
- WO-A-96/39918
- GB-A- 2 301 975
- US-A- 4 676 592
- US-A- 4 905 082
- US-A- 5 528 432
- PATENT ABSTRACTS OF JAPAN vol. 096, no. 002, 29. Februar 1996 & JP 07 287269 A (CANON INC), 31. Oktober 1995
- PATENT ABSTRACTS OF JAPAN vol. 095, no. 003, 28. April 1995 & JP 06 342122 A (OLYMPUS OPTICAL CO LTD), 13. Dezember 1994

## Beschreibung

### Technisches Gebiet

Die Erfindung bezieht sich auf eine Kamera gemäß dem Oberbegriff des Patentanspruches 1.

Gattungsgemäße Kameras werden beispielsweise in der Medizin oder in der Technik zur Untersuchung von Werkstücken eingesetzt. Ein typisches Anwendungsbeispiel für die Verwendung derartiger Kameras in der Medizin sind Dentalkameras, die insbesondere als sogenannte Intraoralkameras benutzt werden. Dentalkameras weisen eine Abbildungsoptik, eine Beleuchtungsoptik, die zumeist aus einer Faseroptik besteht, und einen CCD-Bildaufnehmer auf. Diese Baugruppen sind dabei in einem mehr oder weniger ergonomischen Gehäuse integriert. Weitere Beispiele sind Kameras zur Hautuntersuchung.

### Stand der Technik

Es sind modulare Kamerasysteme bekannt, bei denen beispielsweise durch Wechselobjektive der Anwendungsbereich des Systems erweitert werden kann.

Alle bekannten Systeme sind jedoch mehr oder weniger im Bereich des Praxis-Marketings anzusiedeln, da dem Arzt lediglich die Möglichkeit an die Hand gegeben wird, gemeinsam mit dem Patienten am Bildschirm weitere Behandlungsschritte zu diskutieren.

Der Einsatz der bekannten -auch modularen- gattungsgemäßen Kameras für unterschiedliche Einsatzzwecke ist bislang nicht vorgeschlagen worden.

Ein weiteres Problem bei den bekannten gattungsgemäßen Kameras besteht darin, dass die Kameras entsprechend ihrem Einsatzzweck sehr klein ausgebildet sind. Insbesondere haben die Linsen des Objektivs einen sehr geringen Durchmesser im Bereich von wenigen Millimetern. Damit ist es nicht möglich, spezielle optische Elemente, wie beispielsweise einstellbare Irisblenden oder dergleichen im Abbildungssystem unterzubringen.

Aus der US 4,676,592 ist eine gattungsgemäße Kamera mit den Merkmalen des Oberbegriffs des Patentanspruchs 1, nämlich mit
. optischem System zur Abbildung eines Objektfeldes,
. einen Bildaufnehmer, der das Bild des Objektivs aufnimmt,
. einer Beleuchtungseinheit, deren Licht das Objektfeld des Objektives beleuchtet,
. einer Blende, deren Öffnung verstellbar ist und
. einem Funktionselement, welches bewirkt, dass die Blende und die Fokussierung gleichzeitig verstellbar sind
bekannt.

Aus den US 5,528,432, GB 2,301,975 und US 4,905,082 sind weitere Lösungen bekannt. Auf diese Patentschriften wird ausdrücklich hingewiesen und hinsichtlich der zu entwickelnden Lösung Bezug genommen. Insgesamt weisen diese Lösungen die bereits beschriebenen Nachteile des Standes der Technik auf, die mit der vorliegenden Erfindung beseitigt werden sollen.

### Beschreibung der Erfindung

Der Erfindung liegt die Aufgabe zugrunde, eine gattungsgemäße Kamera derart weiterzubilden, dass ohne gegeneinander verschiebbare Gehäuseteile die Kamera universell und insbesondere als Dentalkamera eingesetzt werden kann.

Eine erfindungsgemäße Lösung dieser Aufgabe ist im Patentanspuch 1 angegeben. Weiterbildungen der Erfindung sind Gegenstand der Unteransprüche.

Bei der Ausbildung der Kamera gemäß dem Oberbegriff des Patentanspruchs 1 wird erfindungsgemäß zunächst, wie aus der gattungsfremden GB 2 301 975 A1 bekannt, eine Beleuchtungseinheit verwendet, deren Licht das Objektfeld des Objektivs beleuchtet. Ferner ist zur Umschaltung des Objektfeldes zwischen kleinem Objektfeld/kleiner Aufnahmeabstand und Übersichtsaufnahme/großer Aufnahmeabstand ein Funktionselement vorgesehen, das gleichzeitig die Fokussierung auf den jeweiligen Aufnahmeabstand und die Blende von einer kleinen Öffnung bei kleinem Aufnahmeabstand auf eine große Öffnung bei großem Aufnahmeabstand und zurück umschaltet, so dass insbesondere die Schärfentiefe dem jeweiligen Einsatzfall angepaßt ist. Damit ist es beispielsweise bei der Dentalkamera möglich, mit ein- und derselben Kamera eine Aufnahme eines Details der Zähne und anschließend eine Aufnahme des Kopfes des Patienten zu machen. Beide Aufnahmen können beispielsweise in einer Bilddatenbank abgelegt werden, so daß der Arzt sich vor Aufruf eines Patienten dessen Gesicht ansehen kann, um den Patienten zuverlässig wieder zu erkennen. Schließlich ist zur Fokussierung mit dem Funktionselement in dem Abbildungslinsensystem eine Linse oder Linsengruppe verschiebbar.

Dabei kann beispielsweise die Apertur des Objektivs mittels einer Aufsteckblende, einer in das Abbildungslinsensystem einsteckbaren Blende oder mittels einer mechanisch oder elektrisch steuerbaren Irisblende geändert werden. Die Aufsteckblende kann insbesondere Bestandteil eines aufsteckbaren, sterilisierbaren Hygieneschutzes sein, der wiederum Bestandteil des Gehäuses sein kann. In den meisten Fällen ist jedoch die Verwendung einer Irisblende bevorzugt, da sie eine einfache Anpassung an die jeweiligen Lichtverhältnisse erlaubt.

Die Fokussierung kann durch Verschieben des Objektivs, bevorzugt jedoch durch Verschieben einer optischen Baugruppe des Objektivs, geändert werden. Ebenso kann die Brennweite durch Verschieben einer Objektivbaugruppe oder durch Austauschen von Linsenbaugruppen geändert werden.

Auch bei der Kamera gemäß Anspruch 1 ist es bevorzugt, wenn zur Anpassung an unterschiedliche Einsatzarten wenigstens ein Parameter mittels eines Funktionselements umgeschaltet wird.

Dabei ist es bevorzugt, wenn zum Umschalten zwischen wenigstens zwei Einsatzarten wenigstens ein optischer Parameter geändert wird. Dieser optische Parameter kann beispielsweise die Apertur des Objektivs, die Fokussierung oder die Brennweite des Objektivs, der Ausblickwinkel der optischen Achse gegenüber dem Gehäuse bei einer sogenannten Schräg-Betrachtung, die Apertur der Beleuchtungsoptik bzw. die Helligkeit des Beleuchtungslichts und/oder das Wellenlängenspektrum des Beleuchtungslichts bzw. der Beleuchtungsoptik sein.

Beispielsweise kann die Brennweite durch Verschieben einer Objektivbaugruppe oder durch Austauschen von Linsenbaugruppen geändert werden.

Das Wellenlängenspektrum kann mittels optischer Filter im Beleuchtungsstrahlengang oder im Abbildungsstrahlengang, durch Wechsel des Beleuchtungsmediums oder durch Änderung des Betriebsmodus des Beleuchtungsmediums, geändert werden.

Zur Änderung der Ausblickrichtung der optischen Achse des Objektivs kann ein bewegliches Prisma oder ein austauschbares Prisma vorgesehen sein. Dabei ist es bevorzugt, wenn entsprechend auch die Blickrichtung der Beleuchtung variiert wird.

Ferner kann die Bildorientierung zwischen seitenrichtig und seitenverkehrt bzw. "gestürzt" geändert werden.

Das erfindungsgemäß vorgesehene Funktionselement, durch dessen Betätigung zwischen unterschiedlichen Einsatzarten der Kamera, beispielsweise einer modularen Dentalkamera umgeschaltet werden kann, kann in den unterschiedlichsten Arten ausgebildet sein.

Beispielsweise können die Funktionselemente mechanisch, z. B. durch eine Translations- oder Rotationsbewegung oder elektrisch arbeiten. Dabei ist es insbesondere bevorzugt, wenn die Funktion mittels einer Sensortaste gesteuert wird, da dann eine Blind-Bedienung möglich wird.

Das Funktionselement kann darüberhinaus einen PC mit einer Standard-Steuer-Software zur Steuerung der einzelnen Funktionen aufweisen.

Ferner ist es möglich, daß das Funktionselement einen Regelkreis aufweist, der Stellgrößen, wie die Fokussierung über einen Sensor erfaßt, so daß dann beispielsweise ein Autofokus-System realisiert werden kann.

Die verschiedenen Einsatzarten können bei einer Dentalkamera beispielsweise Intra- und Extraoralaufnahmen, Röntgenaufnahmen einschließlich Röntgenbild-Digitalisierung, Wurzelkanalaufnahmen und diagnostische Funktionen, wie Kariesfrüherkennung sein.

Wie bereits ausgeführt ist eine bevorzugte Verwendung einer erfindungsgemäßen Kamera die Verwendung als Dentalkamera.

Bei einer Dentalkamera kann mittels des Funktionselements zwischen wenigstens zwei der folgenden Einsatzarten umgeschaltet werden kann:

| | |
|---|---|
| Intraoralaufnahmen, | Extraoralaufnahmen, |
| Röntgenaufnahmen einschlieBlich Röntgenbild Digitalisierung, | Mikroskopadaption, |
| diagnostische Funktionen | Wurzelkanalaufnahmen. |

Weiterhin ist es -wie bereits ausgeführt - möglich, daß eine Aufsteckblende vorgesehen ist, die insbesondere Bestandteil eines aufsteckbaren, sterilisierbaren Hygieneschutzes ist.

### Kurze Beschreibung der Zeichnung

Die Erfindung wird nachstehend anhand eines Ausführungsbeispiels unter Bezugnahme auf die Zeichnung näher beschrieben, in der zeigen:
- Fig. 1: einen Querschnitt durch das Gehäuse eines Ausführungsbeispiels, und
- Fig. 2: die Unteransicht des Gehäuses.

### Darstellung eines Ausführungsbeispiels

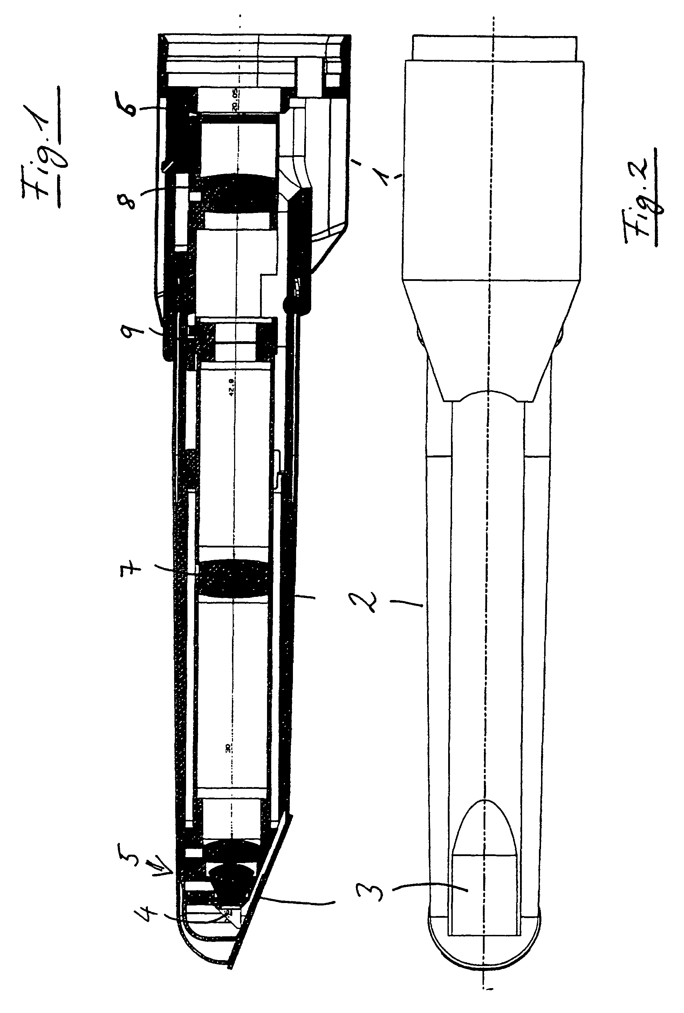

Die in den Figuren 1 und 2 dargestellte Kamera ist ohne Beschränkung des allgemeinen Erfindungsgedankens und der allgemeinen Anwendbarkeit der Erfindung eine Dentalkamera. Die Kamera weist ein Gehäuse auf, das aus einem Grundteil 1, der insbesondere als Griffteil dient, und einem länglichen Ansatz 2 besteht. Der Ansatz 2 erlaubt die Einführung der Kamera in eine Höhle, beispielsweise die Mundhöhle.

Hierzu hat der Ansatz 2 einen Durchmesser, der kleiner ist als der Durchmesser des Grundteils 1. Am vorderen Ende des Ansatzes 2 befindet sich ein Austrittsfenster 3, durch das zum einen das Licht einer nicht näher dargestellten Beleuchtungseinrichtung zur Beleuchtung des (ebenfalls nicht dargestellten) Objektfeldes austritt, und durch das das vom Objektfeld kommende Licht in das Abbildungssystem eintritt.

Das Abbildungssystem weist ein Umlenkprisma 4 auf, das den Abbildungsstrahlengang um einen bestimmten, durch das jeweilige Prisma bestimmten Winkel umlenkt. Im Anschluß an das Prisma 4 ist ein Objektiv 5 vorgesehen, das das Objektfeld in eine nicht näher dargestellte Zwischenbildebene abbildet. Das Zwischenbild wird von einem Abbildungslinsensystem auf einen Bildaufnehmer 6 abgebildet, der beispielsweise ein CCD-Aufnehmer sein kann.

Das Abbildungslinsensystem besteht aus zwei Baugruppen 7 und 8, zwischen denen eine Irisblende 9 oder ein sonstiges optisch wirksames Element angeordnet ist. Eine der beiden Baugruppen 7 bzw. 8 ist zur Fokussierung des Abbildungssystems verschiebbar ausgebildet.

Zur weiteren und insbesondere mechanischen Ausbildung der Kamera wird ausdrücklich auf die zeichnerische Darstellung in den Figuren 1 und 2 Bezug genommen. Deshalb wird auf eine weitergehende Erläuterung verzichtet.

Ausdrücklich wird dabei auf die in Fig. 1 angegebenen Maßangaben Bezug genommen.

## Patentansprüche

1. Kamera mit einem Gehäuse (1,2), mit einem optischen System zur Abbildung eines Objektfeldes, welches mit einem Objektiv (5) mit einem kleinen Linsendurchmesser und mit einem nachgeordneten, eine veränderbare Blende (9) aufweisenden und das Bild des Objektivs (5) auf einen Bildaufnehmer (6) abbildenden Abbildungslinsensystem (7,8) mit einem deutlich größeren Durchmesser versehen ist und mit einer Beleuchtungseinheit, deren Beleuchtungslicht das Objektfeld des Objektivs (5) beleuchtet,
**gekennzeichnet durch** die Kombination folgender Merkmale:
- zur Fokussierung ist in dem Abbildungslinsensystem (7,8) eine Linse oder Linsengruppe verschiebbar,
- zur Umschaltung des abgebildeten Objektfeldes zwischen kleinem Objektfeld /kleiner Aufnahmeabstand und Übersichtsaufname /großer Aufnahmeabstand ist ein Funktionselement vorgesehen, das gleichzeitig die Fokussierung auf den jeweiligen Aufnahmeabstand und zum Zwecke der Einstellungen auf die Schärfentiefe des jeweiligen Einsatzfalles die Blende (9) von einer kleinen Öffnung bei kleinem Aufnahmeabstand auf eine große Öffnung bei großem Aufnahmeabstand und umgekehrt umschaltet.

2. Kamera nach Anspruch 1, **dadurch gekennzeichnet, dass** das Abbildungslinsensystem (7,8) zwei Baugruppen (7; 8) aufweist und dass zur Fokussierung in dem Abbildungslinsensystem (7, 8) eine der Baugruppen (7; 8) verschiebbar ist.

3. Kamera nach Anspruch 1 oder 2, **dadurch gekennzeichnet, dass** die veränderbare Blende (9) zwischen den zwei Baugruppen (7 bzw. 8) des Abbildungslinsensystems (7, 8) angeordnet ist.

4. Kamera nach Anspruch 3, **dadurch gekennzeichnet, dass** die veränderbare Blende eine Irisblene (9) ist.

5. Kamera nach einem der Ansprüche 1 bis 4, **dadurch gekennzeichnet**, das zur Anpassung an unterschiedliche Einsatzarten wenigstens ein weiterer optischer Parameter mittels des Funktionselements umgeschaltet wird.

6. Kamera nach Anspruch 5, **dadurch gekennzeichnet, daß** weitere Parameter
- die Apertur des Objektivs,
- die Fokussierung,
- die Brennweite,
- der Ausblickwinkel der optischen Achse gegenüber dem Gehäuse,
- eine Apertur der Beleuchtungsoptik, und/oder
- die Helligkeit des Beleuchtungslichts und/oder
- das Wellenlängenspektrum der Beleuchtungsoptik sind.

7. Kamera nach einem der Ansprüche 1 bis 6, **dadurch gekennzeichnet, dass** die Brennweite durch Verschieben einer Baugruppe (7 oder 8) des Abbildungslinsensystems oder durch Austauschen von Linsenbaugruppen geändert wird.

8. Kamera nach einem der Ansprüche 1 bis 7, **dadurch gekennzeichnet, dass** das Wellenlängenspektrum mittels optischer Filter im Beleuchtungsstrahlengang oder im Abbildungsstrahlengang durch Wechsel der Beleuchtungseinheit oder durch Änderung des Betriebsmodus des Beleuchtungsmediums geändert wird.

9. Kamera nach einem der Ansprüche 1 bis 8, **dadurch gekennzeichnet, dass** die Änderung des Ausblickwinkels der optischen Achse durch ein bewegliches Prisma (4) oder durch Wechsel des Prismas (4) erfolgt.

10. Kamera nach einem der Ansprüche 1 bis 9, **dadurch gekennzeichnet, dass** das Beleuchtungslicht in der Blickrichtung variierbar ist.

11. Kamera nach einem der Ansprüche 1 bis 10, **dadurch gekennzeichnet**, das die Bildorientierung zwischen seitenrichtig und seitenverkehrt geändert werden kann.

12. Kamera nach einem der Ansprüche 1 bis 11, **dadurch gekennzeichnet**, das das Funktonselement die Funktion mechanisch durch eine Translations- oder Rotationsbewegung einleitet.

13. Kamera nach einem der Ansprüche 1 bis 12, **dadurch gekennzeichnet, dass** das Funktionselement die Funktion elektrisch, beispielsweise mittels einer Sensortaste einleitet.

14. Kamera nach einem der Ansprüche 1 bis 13, **dadurch gekennzeichnet, dass** das Funktionselement einen PC mit einer Steuer-Software aufweist.

15. Kamera nach einem der Ansprüche 1 bis 14, **dadurch gekennzeichnet, dass** das Funktionselement einen Regelkreis aufweist, der Stellgrößen, wie die Fokussierung, über einen Sensor regelt.

16. Verwendung einer Kamera nach einem der Ansprüche 1 bis 15 als Dentalkamera.

17. Kamera nach einem der Ansprüche 1-15 , **dadurch gekennzeichnet, dass** mittels des Funktionselements zwischen wenigstens zwei der folgenden Einsatzarten der kamera als Dentalkamera umgeschaltet werden kann:
| | |
|---|---|
| Intraoralaufnahmen, | Extraoralaufnahmen, |
| | |
| Röntgenaufnahmen einschließlich Röntgenbild-Digitalisierung | Mikroskopadaption, |
| | |
| diagnostische Funktonen | Wurzelkanalaufnahmen. |

18. Kamera nach Anspruch 17, **dadurch gekennzeichnet, dass** eine Aufsteckblende vorgesehen ist.

19. Kamera nach Anspruch 18, **dadurch gekennzeichnet, dass** die Aufsteckblende Bestandteil eines aufsteckbaren, sterilisierbaren Hygieneschutzes ist.

## Claims

1. Camera with a housing (1,2), with an optical system for imaging an object field, which is provided with an objective lens (5) with a small lens diameter and with an imaging lens system (7,8) with a significantly larger diameter disposed behind this, providing a variable diaphragm (9) and mapping the image from the objective lens (5) onto an image pickup (6), and with an illumination unit, of which the illumination light illuminates the object field of the objective lens (5), **characterised by** the combination of the following features:
- for the purpose of focusing, one lens or lens group in the imaging lens system (7,8) is displaceable,
- a functional element is provided in order to switch the mapped object field between a small object field/small pickup distance and an overview image/larger pickup distance, which functional element simultaneously switches the focusing to the relevant pickup distance and, to allow adaptation to the depth of focus of the relevant application, switches the diaphragm (9) from a small aperture with a small pickup distance to a larger aperture with a large pickup distance and vice versa.

2. Camera according to claim 1, **characterised in that** the imaging lens system (7,8) provides two component groups (7;8) and that, for the purpose of focusing, one of the component groups (7;8) in the imaging lens system is displaceable.

3. Camera according to claim 1 or 2, **characterised in that** the variable diaphragm (9) is arranged between the two component groups (7 and/or 8) of the imaging lens system (7,8).

4. Camera according to claim 3, **characterised in that** the variable diaphragm is an iris diaphragm (9).

5. Camera according to any one of claims 1 to 4, **characterised in that** in order to adapt to different modes of operation, at least one further optical parameter is switched by means of the functional element.

6. Camera according to claim 5, **characterised in that** further parameters are:
- the aperture of the objective lens,
- the focusing,
- the focal distance,
- the viewing angle of the optical axis relative to the housing,
- an aperture of the optical illumination system, and/or the brightness of the illumination light and/or
- the wavelength spectrum of the optical illumination system.

7. Camera according to any one of claims 1 to 6, **characterised in that** the focal distance is altered by displacing a component group (7 or 8) of the imaging lens system or by exchanging lens component groups.

8. Camera according to any one of claims 1 to 7, **characterised in that** the wavelength spectrum is altered by means of optical filters in the illumination beam path or in the imaging beam path by exchanging the illumination unit or by altering the mode of operation of the illumination medium.

9. Camera according to any one of claims 1 to 8, **characterised in that** the viewing angle of the optical axis is altered by means of a movable prism (4) or by exchanging the prism (4).

10. Camera according to any one of claims 1 to 9, **characterised in that** the illumination light is variable in the direction of viewing.

11. Camera according to any one of claims 1 to 10, **characterised in that** the image orientation can be changed between true-to-side and lateral inversion.

12. Camera according to any one of claims 1 to 11, **characterised in that** the functional element initiates the function mechanically through a translation movement or a rotational movement.

13. Camera according to any one of claims 1 to 12, **characterised in that** the functional element initiates the function electrically, for example, by means of a sensor key.

14. Camera according to any one of claims 1 to 13, **characterised in** the functional element provides a PC with control software.

15. Camera according to any one of claims 1 to 14, **characterised in that** the functional element provides a control circuit, which controls set parameters such as focusing via a sensor.

16. Use of a camera according to any one of claims 1 to 15 as a dental camera.

17. Camera according to any one of claims 1 to 15, **characterised in that**, as a dental camera, the camera can be switched by means of the functional element between at least two of following modes of operation:
| | |
|---|---|
| Intraoral images, | Extraoral images |
| | |
| X-ray images inducting X-ray image digitisation | Microscope adaptation |
| | |
| Diagnostic functions | Root canal images. |

18. Camera according to claim 17, **characterised in that** a plug-on diaphragm is provided.

19. Camera according to claim 18, **characterised in that** the plug-on diaphragm is a component of a sterilisable, hygienic, plug-on cap.

## Revendications

1. Caméra avec un boîtier (1, 2), avec un système optique pour reproduire un champ de l'objet qui est muni d'un objectif (5) avec un petit diamètre de lentille et d'un système de lentilles de reproduction (7, 8) de diamètre sensiblement plus grand disposé après, présentant un diaphragme (9) variable et reproduisant l'image de l'objectif (5) sur un appareil de prise de vue (6) et avec une unité d'éclairage dont la lumière d'éclairage éclaire le champ de l'objet de l'objectif (5), **caractérisée par** la combinaison des caractéristiques suivantes :
- pour la mise au point, une lentille ou un groupe de lentilles peut se déplacer dans le système de lentilles de reproduction (7, 8),
- pour commuter le champ de l'objet reproduit entre petit champ de l'objet/courte distance de prise de vue et prise de vue d'ensemble/grande distance de prise de vue, il est prévu un élément de fonction qui commute simultanément la mise au point sur la distance de prise de vue correspondante et le diaphragme (9) d'une petite ouverture en cas de courte distance de prise de vue à une grande ouverture en cas de grande distance de prise de vue et inversement, afin de faire les réglages à la profondeur de champ du cas d'utilisation correspondant

2. Caméra selon la revendication 1, **caractérisée en ce que** le système de lentilles de reproduction (7, 8) présente deux groupes (7 ; 8) et qu'un des groupes (7 ; 8) peut se déplacer dans le système de lentilles de reproduction (7, 8) pour la mise au point.

3. Caméra selon la revendication 1 ou 2, **caractérisée en ce que** le diaphragme variable (9) est disposé entre les deux groupes (7 et 8) du système de lentilles de reproduction (7, 8).

4. Caméra selon la revendication 3, **caractérisée en ce que** le diaphragme variable est un diaphragme à iris (9).

5. Caméra selon l'une des revendications 1 à 4, **caractérisée en ce que**, pour l'adaptation à des modes d'utilisation différents, au moins un autre paramètre optique est commuté au moyen de l'élément de fonction.

6. Caméra selon la revendication 5, **caractérisée en ce que** d'autres paramètres sont :
- l'ouverture de l'objectif,
- la mise au point,
- la distance focale,
- l'angle de vue de l'axe optique par rapport au boîtier,
- une ouverture de l'optique d'éclairage et/ou
- la luminosité de la lumière d'éclairage et/ou le
- spectre de longueur d'onde de l'optique d'éclairage.

7. Caméra selon l'une des revendications 1 à 6, **caractérisée en ce que** la distance focale est modifiée par déplacement d'un groupe (7 ou 8) du système de lentilles de reproduction ou par échange de groupes de lentilles.

8. Caméra selon l'une des revendications 1 à 7, **caractérisée en ce que** le spectre de longueur d'onde est modifié au moyen de filtres optiques dans la trajectoire des rayons d'éclairage ou dans la trajectoire des rayons de reproduction par changement de l'unité d'éclairage ou par modification du mode de fonctionnement du moyen d'éclairage.

9. Caméra selon l'une des revendications 1 à 8, **caractérisée en ce que** la variation de l'angle de vue de l'axe optique est effectuée par un prisme mobile (4) ou par remplacement du prisme (4).

10. Caméra selon l'une des revendications 1 à 9, **caractérisée en ce que** la lumière d'éclairage est variable dans la direction du regard.

11. Caméra selon l'une des revendications 1 à 10, **caractérisée en ce que** l'orientation de l'image peut être modiflée entre endroit et envers.

12. Caméra selon l'une des revendications 1 à 11, **caractérisée en ce que** l'élément de fonction initie la fonction mécaniquement par un mouvement de translation ou de rotation.

13. Caméra selon l'une des revendications 1 à 12, **caractérisée en ce que** l'élément de fonction initie la fonction de manière électrique, au moyen d'une touche à effleurement, par exemple.

14. Caméra selon l'une des revendications 1 à 13, **caractérisée en ce que** l'élément de fonction présente un PC avec un logiciel de commande.

15. Caméra selon l'une des revendications 1 à 14, **caractérisée en ce que** l'élément de fonction présente un circuit de régulation, qui règle par l'intermédiaire d'un capteur des valeurs de réglages telles que la mise au point.

16. Utilisation d'une caméra selon l'une des revendications 1 à 15 sous la forme d'une caméra dentaire.

17. Caméra selon l'une des revendications 1 à 15, **caractérisée en ce qu'**on peut commuter au moyen de l'élément de fonction entre au moins deux des modes d'utilisation suivants de la caméra en tant que caméra dentaire :
| | |
|---|---|
| Prises de vues intraorales | Prises de vues extraorales, |
| | |
| Radiographies y compris digitalisation de l'image radio | Adaptation de microscope, |
| | |
| Fonctions diagnostiques | Prises de vue de canal de racine. |

18. Caméra selon la revendication 17, **caractérisée en ce qu'**il est prévu un diaphragme à emboîter.

19. Caméra selon la revendication 18, **caractérisée en ce que** le diaphragme à emboîter fait partie d'une protection hygiénique stérilisable, pouvant être emboîtée.
